(19) **Europäisches Patentamt / European Patent Office / Office européen des brevets**

(11) **EP 4 443 145 A1**

(12) # EUROPEAN PATENT APPLICATION
published in accordance with Art. 153(4) EPC

(43) Date of publication:
**09.10.2024 Bulletin 2024/41**

(21) Application number: **22901385.9**

(22) Date of filing: **30.11.2022**

(51) International Patent Classification (IPC):
**G01N 22/00** *(2006.01)* **G01N 22/04** *(2006.01)*

(52) Cooperative Patent Classification (CPC):
**G01N 22/00; G01N 22/04**

(86) International application number:
**PCT/JP2022/044265**

(87) International publication number:
**WO 2023/100954 (08.06.2023 Gazette 2023/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.12.2021 JP 2021196068**

(71) Applicants:
• **SUMITOMO METAL MINING CO., LTD.
Tokyo
105-8716 (JP)**
• **National University Corporation Chiba University
Chiba-shi, Chiba 263-8522 (JP)**

(72) Inventors:
• **NAITO, Motoyuki
Niihama-shi, Ehime 792-0001 (JP)**
• **SRI SUMANTYO, Josaphat Tetuko
Chiba-shi, Chiba 263-8522 (JP)**
• **TAKAHASHI, Ayaka
Chiba-shi, Chiba 263-8522 (JP)**

(74) Representative: **Jones, Nicholas Andrew
Withers & Rogers LLP
2 London Bridge
London SE1 9RA (GB)**

(54) **METHOD FOR MEASURING STATE OF SUBSTANCE AND DEVICE FOR MEASURING STATE OF SUBSTANCE**

(57)    A method for measuring a state of a substance, includes an irradiating process that irradiates an electromagnetic wave with respect to the substance inside a closed space, a receiving process that receives the electromagnetic wave, and a data processing process that performs a data processing on the electromagnetic wave received by the receiving process, wherein the irradiating process uses a chirped pulse wave as the electromagnetic wave.

FIG.1

10

START → IRRADIATING PROCESS ~S11 → RECEIVING PROCESS ~S12 → DATA PROCESSING PROCESS ~S13 → END

EP 4 443 145 A1

**Description**

TECHNICAL FIELD

The present invention relates to methods for measuring states of substances, and devices for measuring states of substances.

BACKGROUND ART

Various furnaces, such as a flash furnace, a kiln, or the like as disclosed in Patent Document 1, for example, are conventionally used for refining, synthesis of various materials, or the like.
When the furnace is used for refining, synthesis of various materials, or the like, there are demands to accurately grasp a state of a substance inside the furnace from a viewpoint of optimizing a reaction condition in the furnace and increasing productivity, and an infrared camera or the like is conventionally provided inside the furnace.

PRIOR ART DOCUMENTS

PATENT DOCUMENTS

Patent Document 1: Japanese Laid-Open Patent Publication No. 2019-183202

DISCLOSURE OF THE INVENTION

PROBLEM TO BE SOLVED BY THE INVENTION

However, gas, dust, or the like may be generated in a closed space, such as the inside of the furnace or the like, and the infrared camera cannot accurately measure the state of the substance inside the furnace due to the influence of the gas or the like.
In view of the problem of the conventional technique described above, one object according to an aspect of the present invention is to provide a method for measuring a state of a substance inside a closed space.

MEANS OF SOLVING THE PROBLEM

In order to solve the problem described above, one aspect of the present invention provides a method for measuring a state of a substance, including an irradiating process that irradiates an electromagnetic wave with respect to the substance inside a closed space; a receiving process that receives the electromagnetic wave; and a data processing process that performs a data processing on the electromagnetic wave received by the receiving process, wherein the irradiating process uses a chirped pulse wave as the electromagnetic wave.

EFFECTS OF THE INVENTION

According to one aspect of the present invention, it is possible to provide a method for measuring a state of a substance, which is capable of measuring the state of the substance inside a closed space.

BRIEF DESCRIPTION OF THE DRAWINGS

[FIG. 1] FIG. 1 is a flow chart of a method for measuring a state of a substance according to one embodiment of the present invention.
[FIG. 2] FIG. 2 is a schematic diagram of a device for measuring a state of a substance according to one embodiment of the present invention.
[FIG. 3] FIG. 3 is a diagram for explaining a chirped pulse wave.
[FIG. 4A] FIG. 4A is a diagram for explaining the chirped pulse wave.
[FIG. 4B] FIG. 4B is a diagram for explaining the chirped pulse wave.
[FIG. 4C] FIG. 4C is a diagram for explaining the chirped pulse wave.
[FIG. 4D] FIG. 4D is a diagram for explaining the chirped pulse wave.
[FIG. 4E] FIG. 4E is a diagram for explaining the chirped pulse wave.

[FIG. 5A] FIG. 5A is a diagram for explaining an experimental state of an exemplary implementation 1.
[FIG. 5B] FIG. 5B is a diagram for explaining the experimental state of the exemplary implementation 1.
[FIG. 6A] FIG. 6A is a diagram for explaining an experimental condition of the exemplary implementation 1.
[FIG. 6B] FIG. 6B is a diagram for explaining the experimental condition of the exemplary implementation 1.
[FIG. 7A] FIG. 7A illustrates an analysis result of an electromagnetic wave received in a receiving process of the exemplary implementation 1.
[FIG. 7B] FIG. 7B illustrates the analysis result of the electromagnetic wave received in the receiving process of the exemplary implementation 1.
[FIG. 7C] FIG. 7C illustrates the analysis result of the electromagnetic wave received in the receiving process of the exemplary implementation 1.
[FIG. 8] FIG. 8 illustrates an evaluation result of an exemplary implementation 2.
[FIG. 9A] FIG. 9A illustrates the evaluation result of an exemplary implementation 3-1.
[FIG. 9B] FIG. 9B illustrates the evaluation result of the exemplary implementation 3-1.
[FIG. 9C] FIG. 9C illustrates the evaluation result of the exemplary implementation 3-1.
[FIG. 10A] FIG. 10A illustrates the evaluation result of an exemplary implementation 3-2.
[FIG. 10B] FIG. 10B illustrates the evaluation result of the exemplary implementation 3-2.
[FIG. 10C] FIG. 10C illustrates the evaluation result of the exemplary implementation 3-2.

MODE OF CARRYING OUT THE INVENTION

Hereinafter, embodiments for carrying out the present invention will be described with reference to the drawings, but the present invention is not limited to the following embodiments, and various modifications and substitutions can be made to the following embodiments without departing from the scope of the present invention.

[Method and Device for Measuring State of Substance]

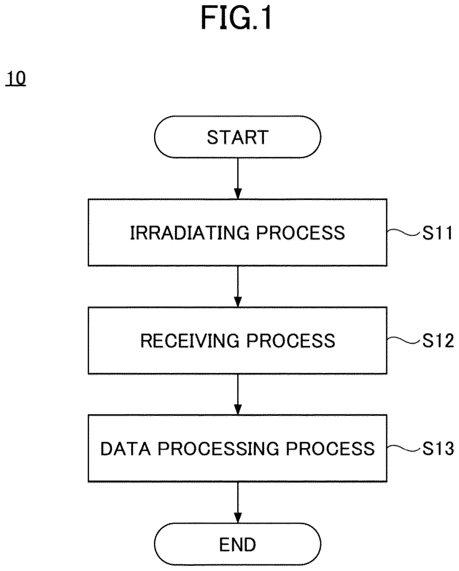

A method for measuring a state of a substance according to the present embodiment can be performed in accordance with a flow chart 10 illustrated in FIG. 1, and can include an irradiating process (step S11), a receiving process (step S12), and a data processing process (step S13) described hereunder.

The irradiating process (step S11) can irradiate an electromagnetic wave on a substance inside a closed space.

The receiving process (step S12) can receive the electromagnetic wave.

The data processing process (step S13) can process the electromagnetic wave received by the receiving process (step S12).

In the irradiating process (step S11), a chirped pulse wave can be used as the electromagnetic wave.

A device for measuring a state of a substance according to the present embodiment will be described below, and the method for measuring the state of the substance according to the present embodiment will be described thereafter. The device for measuring the state of the substance according to the present embodiment can be suitably used in the method for measuring the state of the substance according to the present embodiment.

(1) Device for Measuring State of Substance

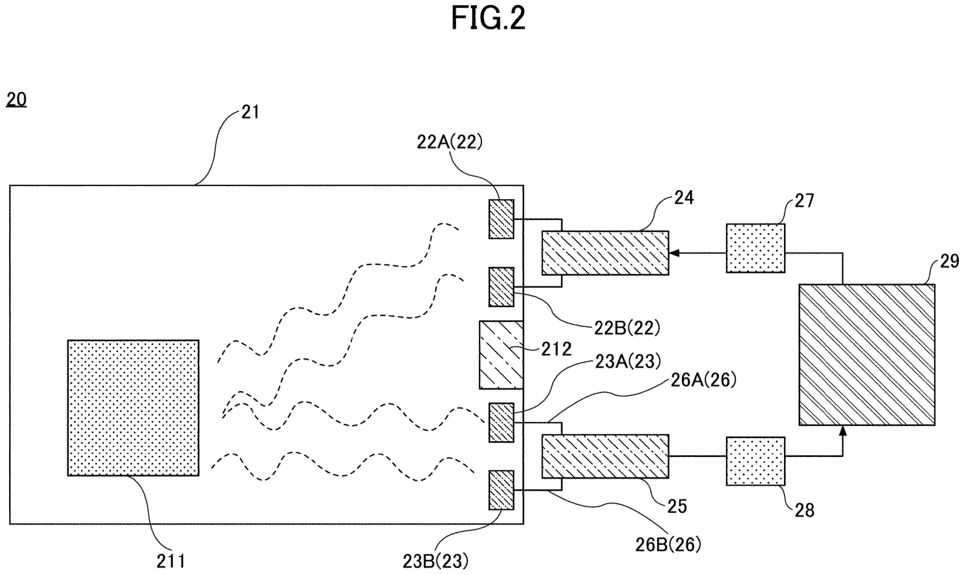

FIG. 2 is a schematic diagram of the device for measuring the state of the substance, which can be suitably used when performing the method for measuring the state of the substance according to the present embodiment.

A device 20 for measuring the state of the substance according to the present embodiment illustrated in FIG. 2 can include a closed space 21, a transmitting section 22 that irradiates an electromagnetic wave with respect to a substance 211 in the closed space 21, a receiving section 23 that receives the electromagnetic wave, and an analyzer 29. The analyzer 29 can perform a data processing on data of the electromagnetic wave received by the receiving section 23.

Each part of the device will be described below.

(Closed Space)

The closed space 21 may be the inside of various heating furnaces, such as a flash furnace, a kiln, or the like, or the inside of internal combustion engines, that is, engines or the like, for example. The inside of the heating furnace or the internal combustion engine refers to a region where a raw material or fuel is supplied and a reaction or combustion is performed, and is a region separated from other regions by a partition wall.

The closed space may have a gap or the like, formed in the partition wall that partitions the closed space, and provided for a pipe for supplying the raw material or fuel into the closed space, a pipe for discharging a product or exhaust gas, or a member having a position that is displaced as required, such as a piston or the like. For this reason, the closed space may be partitioned by the partition wall, and does not have to be a completely sealed space.

The state of the substance 211, which is an object to be measured, is not particularly limited, and may be in either a solid or liquid state. In addition, the solid state and the liquid state may coexist, or a cavity may be present inside the substance 211.

In FIG. 2, the substance 211, which is the object to be measured, is schematically illustrated by one square, but the substance 211 may be a powder or the like including a plurality of particles, or a group of droplets or the like, and a portion or all of the particles or the like may be dissolved and include a liquid portion.

An accessory device, such as a heating means or the like, such as a heater or the like for treating the substance 211, may be provided inside or outside the closed space 21.

(Transmitting Section)

The transmitting section 22 can be configured to be able to irradiate electromagnetic wave with respect to the substance 211 in the closed space 21, and can include an antenna, for example. A shape, type, number, or the like of the antenna are not particularly limited, and can be selected according to an environment of the closed space, the characteristics of the electromagnetic wave used, or the like.

When evaluating the state of the substance inside the closed space, a method is conceivable in which a transmitting section irradiates a rectangular pulse wave with respect to the substance, for example, and a receiving section receives and analyzes a reflection signal reflected by each substance.

However, in a case where the state of the substance inside the closed space is measured using the electromagnetic wave, a transmitting section, the substance to be measured, and a receiving section are arranged within a specific limited region. For this reason, in a case where the states of a plurality of substances are to be measured, for example, there is no large difference in distances between the transmitting section and the plurality of substances to be measured and no large difference in distances between the receiving section and the plurality of substances to be measured. As a result, there is little difference in times when the reflected signals reflected by the plurality of substances to be measured reach the receiving section after the plurality of substances to be measured are irradiated with the light from the transmitting section, and the reflected signals overlap, thereby possibly deteriorating a resolution.

As a means for solving such a problem, it is conceivable to narrow the pulse width, so that the reflected signals can be separated. However, when the pulse width is narrowed, a transmission power when transmitting the electromagnetic wave from the transmitting section decreases, and a signal-to-noise ratio (S/N ratio) may deteriorate.

Accordingly, the transmitting section preferably uses a chirped pulse wave as the electromagnetic wave. That is, the transmitting section preferably irradiates (transmits) the chirped pulse wave.

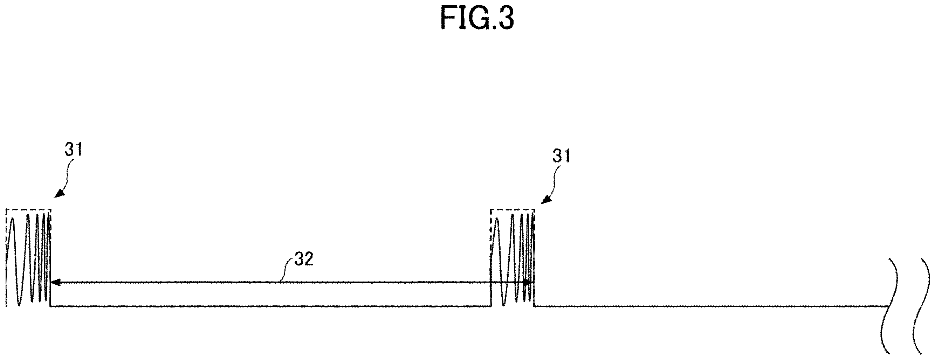

The chirped pulse wave may have a waveform illustrated in FIG. 3, for example. That is, the chirped pulse wave has a waveform in which a pulse signal 31, having a frequency that varies with time, appears at a predetermined pulse repetition interval 32.

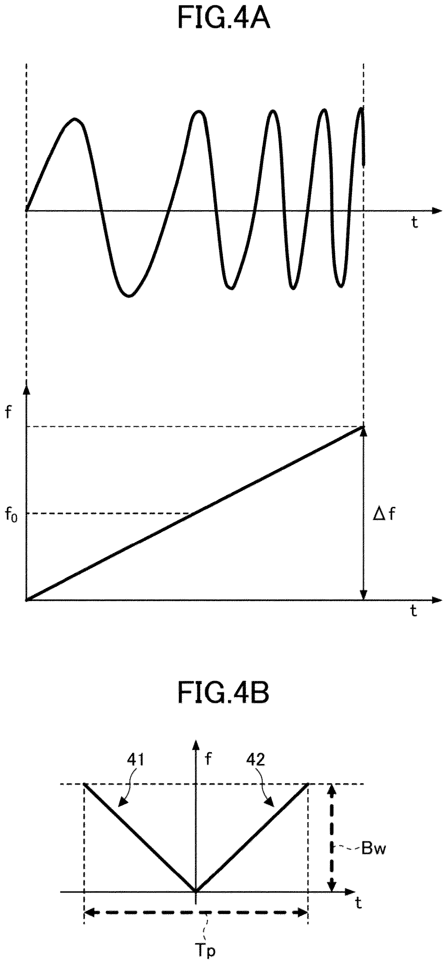

The pulse signal 31 of the chirped pulse wave is a wave in which the frequency f varies proportionally to time t centered around a predetermined frequency $f_0$ as illustrated in FIG. 4A, for example. The frequency f of the pulse signal wave of the chirped pulse wave is not limited to the case where the frequency f varies proportionally to the time t, and can be expressed by an arbitrary function having a correlation with the time t.

The frequency of the pulse signal of the chirped pulse wave can be expressed by the following Formula (1).
[Math. 1]

$$f = f_0 + \alpha t \quad \cdots (1)$$

In addition, an output waveform $g_s(t)$ of the pulse signal of the chirped pulse wave can be expressed by the following Formula (2) or Formula (3).
[Math. 2]

$$g_s(t) = \exp[2\pi i(f_0 + \alpha t)t] \quad \cdots (2)$$

[Math. 3]

$$g_s(t) = W(t) \times \exp[2\pi i(f_0 + \alpha t)t] \quad \cdots (3)$$

In the Formulas (1) through (3) described above, $\alpha$ can be an arbitrarily determined constant, or a function of the time t. In the Formulas (2) and (3) described above, i denotes an imaginary unit.

In the Formula (3) described above, W(t) denotes a weight function, and the resolution can be increased by further manipulating a power distribution of the basic chirped pulse waveform, and the resolution of the obtained signal image can be increased, for example. Specifically, in addition to a rectangular window, a Gaussian window, a Hamming window, a Kaiser window, or the like, a multi-level chirp compression represented by a repetitive chirped pulse compression using a regressive chirped pulse or the like can be effective.

A coefficient of the Kaiser window can be expressed by the following Formula (4), for example.

[Math. 4]

$$w(t) = \frac{I_0\left(\beta\sqrt{1 - \left(\frac{t - Tp/2}{Tp/2}\right)^2}\right)}{I_0(\beta)} \quad \cdots (4)$$

In the Formula (4), $I_0$ denotes a zero order first type modified Bessel function, Tp denotes a width (length) of the Kaiser window, t denotes an arbitrary real number satisfying a relationship $-Tp/2 <= t <= Tp/2$, and $\beta$ denotes an arbitrary positive real number.

A lower part of FIG. 4A illustrates a relationship between the time t and the frequency f of the pulse signal 31, and indicates that the frequency varies by $\Delta f$ centered around the predetermined frequency $f_0$.

Further, by setting $\alpha$ in the expressions of an output waveform of the pulse signal of the chirped pulse wave expressed by the Formula (2) or the Formula (3) to a function of the time t, a chirped pulse wave generated by a multi-level chirp modulation can be obtained.

In this case, $\alpha(t)$ as the function of the time t can be expressed by the following Formula (5) and Formula (6). $\gamma(t)$ in the Formula (5) is expressed by the Formula (6). Tp in the Formula (6) denotes the width of the pulse signal of the chirped pulse wave, and t denotes the time, m can be arbitrarily selected, and a chirp rate of the multi-level modulation can be adjusted thereby.

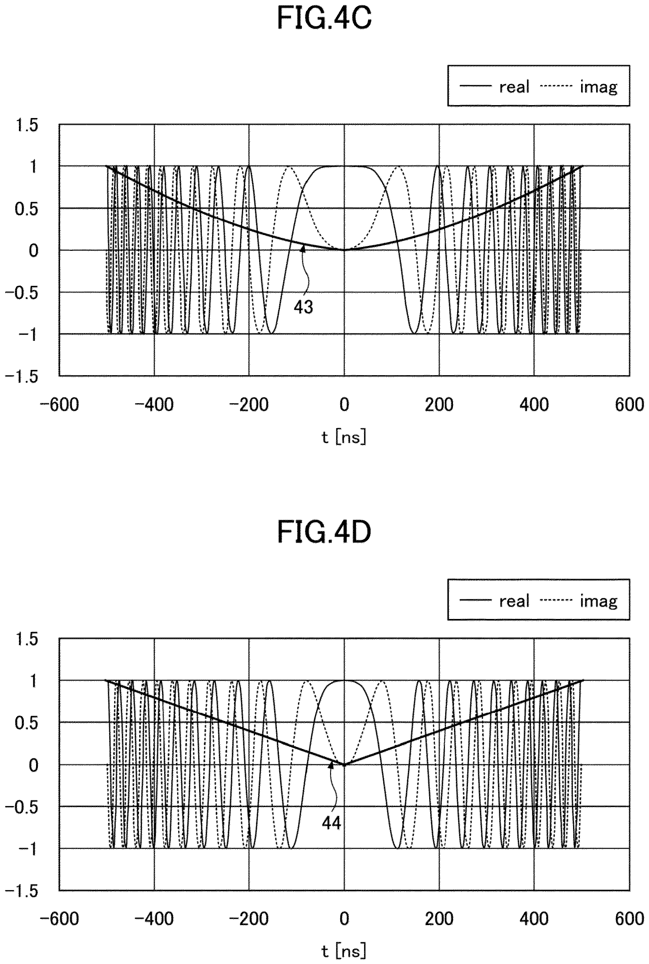

In the Formula (6), t denotes an absolute because the pulse signal of a symmetric chirped pulse wave is assumed using t=0 as an axis of symmetry, as in FIG. 4C through FIG. 4E which will be described later. In this case, the time t also denotes an absolute value in the Formulas (1) through (3) described above.

[Math. 5]

$$\alpha_m(t) = \gamma(t) \cdot \alpha \quad \cdots (5)$$

[Math. 6]

$$\gamma(t) = \left(\frac{|t|}{Tp/2}\right)^m \quad \cdots (6)$$

As illustrated in FIG. 4B, the frequency of the pulse signal wave of the chirped pulse wave decreases and increases according to the time t during the width Tp of the pulse signal of the chirped pulse wave, and a maximum frequency is assumed to be Bw. A section 41 in which the frequency decreases may be referred to as a Down-Chirp, and a section 42 in which the frequency increases may be referred to as an Up-Chirp.

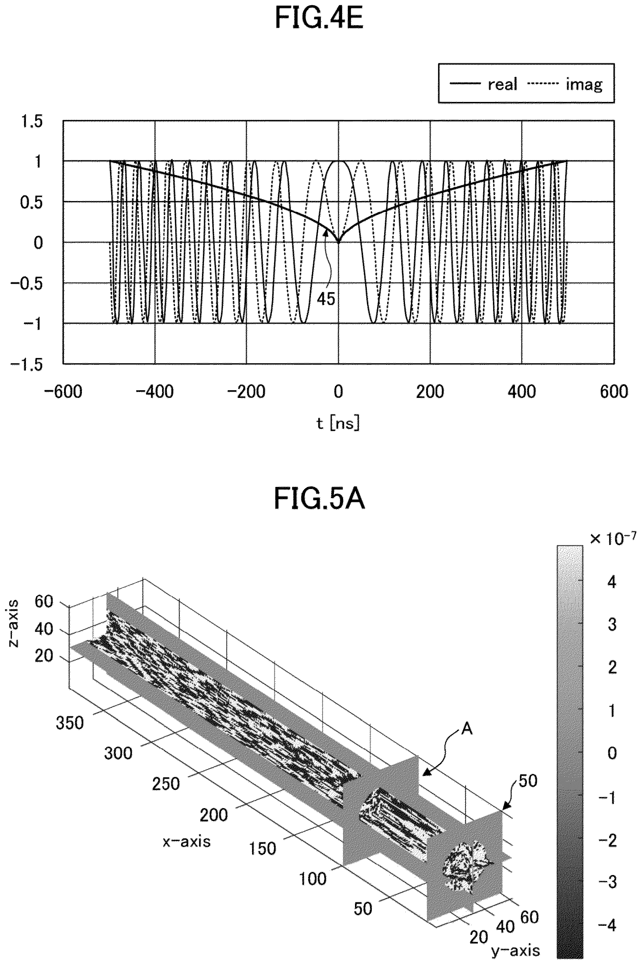

In a case where m in the Formula (6) is 1.5, the frequency of the pulse signal of the chirped pulse wave varies with respect to the time t along a curve 43 similar to a parabola, as illustrated in FIG. 4C. FIG. 4C illustrates the real part (real) and the imaginary part (imag) separately, and the same applies to FIG. 4D and FIG. 4E which will be explained below.

In a case where m in the Formula (5) is 1.0, the frequency of the pulse signal of the chirped pulse wave varies along a straight line 44 with respect to the time t, as illustrated in FIG. 4D.

In a case where m in the Formula (5) is 0.6, the frequency of the pulse signal of the chirped pulse wave varies along a curve 45 similar to a parabola, as illustrated in FIG. 4E.

By using the chirped pulse wave described above, even in a case where the pulse width is narrowed, it is possible to reduce deterioration of the S/N ratio. For this reason, it is possible to increase the S/N ratio while increasing the resolution of the measurement result of the state of the substance.

The pulse repetition interval 32 is not particularly limited, but can be adjusted and selected so that the next pulse signal 31 is irradiated when the pulse signal is irradiated on the substance 211 and a magnitude of multiple scattering waves generated due to scattering becomes less than or equal to a predetermined value.

The transmitting section 22 preferably uses a chirped pulse wave generated by the multi-level chirp modulation as the electromagnetic wave from a viewpoint of increasing the resolution of the device for measuring the state of the substance according to the present embodiment.

The transmitting section 22 preferably uses a circularly polarized wave as the electromagnetic wave. Because the substance 211 to be measured usually has a three-dimensional shape, the use of the circularly polarized wave also makes it possible to perform the measurement of a particle size distribution or the like of the substance with a particularly high accuracy.

Accordingly, the transmitting section 22 may include a first transmitting section 22A which is an antenna for transmitting a right-handed circularly polarized wave, and a second transmitting section 22B which is an antenna for transmitting a left-handed circularly polarized wave, for example.

In the case where the transmitting section 22 uses the circularly polarized wave as the electromagnetic wave, the transmitting section 22 may be configured to include only one of the first transmitting section 22A and the second transmitting section 22B as an antenna and use only one of the right-handed circularly polarized wave and the left-handed circularly polarized wave. Further, an antenna capable of switching between right rotation and left rotation or a combination of right rotation and left rotation may be used for transmission and reception. By using the antenna capable of switching between the right rotation and the left rotation or the combination thereof in the transmitting section or the receiving section, it is possible to obtain a larger variety of information related to the state of the substance.

Phases of the chirped pulse wave of the electromagnetic wave irradiated from the first transmitting section 22A and the electromagnetic wave irradiated from the second transmitting section 22B are preferably shifted from each other.

A frequency range of the electromagnetic wave irradiated from the transmitting section 22 is not particularly limited, and can be arbitrarily selected according to a type of the substance which is the object to be measured, a material used for the partition wall forming the closed space 21, or the like. An electromagnetic wave of 300 MHz or higher and 40 GHz or lower can be suitably used as the electromagnetic wave described above.

(Receiving Section)

The receiving section 23 can receive the electromagnetic wave irradiated from the transmitting section 22. Specifically, the receiving section 23 can receive the reflection signal of the electromagnetic wave irradiated from the transmitting section 22 and reflected by the substance 211.

The receiving section 23 can be configured to be able to irradiate the electromagnetic wave, and can include an antenna, for example. A shape, type, number, or the like of the antenna are not particularly limited, and can be selected according to the environment of the closed space, the characteristics of the electromagnetic wave used, or the like.

The configuration of the receiving section 23 is not particularly limited, and the receiving section 23 may have a configuration according to the transmitting section 22 or the like. For example, in the case where the transmitting section 22 includes the first transmitting section 22A which is the antenna for transmitting the right-handed circularly polarized wave and the second transmitting section 22B which is the antenna for transmitting the left-handed circularly polarized wave as described above, the receiving section 23 may include a first receiving section 23A and a second receiving section 23B. The first receiving section 23A can be an antenna that receives the right-handed circularly polarized wave, for example, and the second receiving section 23B can be an antenna that receives the left-handed circularly polarized wave, for example.

(Analyzer)

The analyzer 29 can perform the data processing on the data of the electromagnetic wave received by the receiving section. The data processing performed by the analyzer 29 enables data on the state of the substance to be acquired.

Contents of the data processing performed by the analyzer 29 are not particularly limited, and the data processing can perform a process according to the data on the state of the substance to be acquired or the like. For example, the data of the electromagnetic wave received by the receiving section can be separated into a real part and an imaginary part, and the data processing can perform a process according to the data on the state of the substance to be acquired.

Specifically, the analyzer 29 can perform a chirp compression on the data of the electromagnetic wave, for example.

A transmitting wave transmitted from the transmitting section 22 is denoted by $g_S(t)$, and a received wave received by the receiving section 23 is denoted by $g_R(t)$. Further, the transmitting wave is set to $g_S(t) = \exp[2\pi I(f0 + \alpha t)t]$ or the like as in the Formula (2) described above.

Under the above conditions, a cross correlation between the transmitting wave and the received wave can be expressed by the following Formula (7).

[Math. 7]

$$C(T) = \overline{g_R(t) \times g_s(t+T)} = \int g_R(t)g_s^*(t+T)dt \quad \cdots (7)$$

When a Fourier transform of the Formula (7) described above is denoted by F, the following Formula (8) can be obtained.
[Math. 8]

$$F(C) = F(g_R) \cdot F^*(g_S) \quad \cdots (8)$$

Accordingly, by using the Fourier transform, a sum of products operation in the time domain is expressed by a product in the frequency space, and the amount of calculation can be reduced. Hence, in the analyzer 29, it is preferable to calculate the Fourier transform of the correlation function in the frequency space, and perform an inverse Fourier transform in order to reduce the amount of calculation. In addition, by a power distribution operation or the like using a window function, it is possible to improve the resolution and the S/N ratio (signal-to-noise ratio).

As described above, the electromagnetic wave irradiated from the transmitting section 22 is more preferably a chirped pulse wave generated by the multi-level chirp modulation. In a case where the chirped pulse wave generated by the multi-level chirp modulation is irradiated from the transmitting section 22, the multi-level chirp compression can be performed in the analyzer 29 according to the number of times of the chirp modulation is performed, the chirp rate, or the like. By performing the multi-level chirp compression, it is possible to increase the resolution.

The device for measuring the state of the substance according to the present embodiment performs the measurement on the substance inside the closed space 21, and the receiving section 23 also receives the electromagnetic wave reflected by the partition wall for forming the closed space 21, including noise, specifically, speckle noise, for example. For this reason, the analyzer 29 preferably performs a process of removing the noise before the data processing described above. The method for removing noise is not particularly limited, and a correlation formula of the noise in the device for measuring the state of the substance may be obtained by the least squares method, and the noise can be removed by subtracting the correlation formula from the received data, for example.

From the results obtained by the data processing described above, a further data processing may be performed according to data required for the state of the substance 211. According to the device for measuring the state of the substance according to the present embodiment, data, such as the particle size distribution of the substance 211, a distribution state of the substance 211 inside the closed space 21, a composition of the substance 211, a moisture percentage of the substance 211, or the like can be acquired as the state of the substance 211. For this reason, a further data processing may be performed according to the data or the like required for the state of the substance 211 described above, for example.

The analyzer 29 can also calculate the waveform of the electromagnetic wave output from a transmitter 24, and output the waveform to the transmitter 24. As described above, the transmitter 24 can irradiate the chirped pulse wave, and the analyzer 29 can determine the waveform to be output according to the condition or the likes of the chirped pulse wave. A waveform determination circuit may be provided separately from the analyzer, and the waveform determination circuit may calculate the waveform of the electromagnetic wave output from the transmitter 24 or the like.

The analyzer 29 can be configured to include a personal computer (PC) or the like. For this reason, the analyzer 29 can be configured by software and hardware in cooperation with each other by executing a program prestored in a central processing unit (CPU) of an information processing apparatus, such as a personal computer or the like.

The analyzer 29 may include a CPU which is an arithmetic processing unit, a random access memory (RAM) or a read only memory (ROM) which is a main storage device, an auxiliary storage device, an input-output interface, a display device which is an output device, or the like. The CPU, the main storage device, the auxiliary storage device, the input-output interface, and the output device included in the analyzer 29 can be connected to one another via a bus. All of the constituent elements of the analyzer 29 described above are not necessarily accommodated in the same housing, and the auxiliary storage device and the display device may be provided outside the housing, for example. The auxiliary storage device is a storage device, such as a solid state drive (SSD), a hard disk drive (HDD), or the like.

Examples of the input-output interface include a user interface, such as a touchscreen panel, a keyboard, a mouse, a display screen, an operation button, or the like, and a wired or wireless communication interface for exchanging control signals and data with an external device.

For example, in a case where the closed space 21 is a firing furnace or the like, the results of measurement made by the device for measuring the state of the substance according to the present embodiment can be output to a controller of the firing furnace via the input-output interface.

Examples of the output device include a display which is a display device, a printer, or the like. The results of the measurement made by the device for measuring the state of the substance according to the present embodiment can be output to the output device.

The device for measuring the state of the substance according to the present embodiment can further include various other constituent elements, as required.

(Transmitter, Receiver, D/A Converter, and A/D Converter)

As described above, the analyzer 29 can determine the waveform of the chirped pulse wave to be irradiated from the transmitting section 22, and perform the data processing on the data of the electromagnetic wave received by the receiving section 23.

As illustrated in FIG. 2, for example, a digital-to-analog (D/A) converter 27 and the transmitter 24 may be disposed between the analyzer 29 and the transmitting section 22. The D/A converter 27 can convert the data of the chirped pulse wave determined by the analyzer 29, from digital data into analog data. The transmitter 24 can amplify and modulate the chirped pulse wave as required, based on the data of the chirped pulse wave acquired from the D/A converter 27, and can irradiate the chirped pulse wave from the transmitting section 22.

For example, a receiver 25 and an analog-to-digital (A/D) converter 28 may be arranged between the receiving section 23 and the analyzer 29. The receiver 25 can convert the signal received by the receiving section 23 into an electric signal, for example. Further, the A/D converter 28 can convert the analog data obtained by the receiving section 23 into digital data, and supply the digital data to the analyzer 29.

The analyzer 29 may be connected to the transmitter 24 and the receiver 25 via a control line or the like that are not illustrated, as required, and the analyzer 29 can be configured to control the operations of the transmitter 24 and the receiver 25.

Instead of separately providing the transmitter 24, the receiver 25, the D/A converter 27, and the A/D converter 28, and the analyzer 29, the transmitting section 22, and the receiving section 23 may be configured to perform some or all of the functions of the transmitter 24, the receiver 25, the D/A converter 27, and the A/D converter 28.

(Delay Line)

The device for measuring the state of the substance according to the present embodiment is a device for measuring the state of the substance inside the closed space 21. For this reason, there is almost no time difference between a signal (scattered wave) which is irradiated from the transmitting section 22, reflected by each substance 211 to be measured, and received by the receiving section 23, and a signal (direct wave) which is directly received from the transmitting section 22 by the receiving section 23.

As a result, in some cases, it may be difficult to separate the scattered wave and the direct wave.

Although it is conceivable to solve the problem described above by selecting the pulse width according to the size of the closed space 21 or the like depending on the size of the closed space 21, in some cases, it may not be possible to sufficiently separate the scattered wave and the direct wave by merely selecting the pulse width.

Hence, the device for measuring the state of the substance according to the present embodiment preferably has a delay line 26 between the receiving section 23, which is an antenna, and the receiver 25 which converts the signal received by the receiving section 23 into an electrical signal. By providing the delay line 26, it is possible to secure a sufficient time after the electromagnetic wave is transmitted from the transmitting section 22 until the electromagnetic wave is converted into the electric signal by the receiver 25. For this reason, it is possible to duplicate a situation similar to a case where the measurement is performed in a space larger than the closed space 21 in which the measurement is being performed, so that the scattered wave and the direct wave described above can easily be separated.

A delay line having a delay time greater than or equal to the pulse width of the chirped pulse wave transmitted from the transmitting section 22 is preferably used as the delay line.

As described above, in the case where the first receiving section 23A and the second receiving section 23B are provided, a delay line may be provided between each receiving section and the receiver 25, and a first delay line 26A and a second delay line 26B may be provided as illustrated in FIG. 2.

An extent or the like of delay by the delay line 26 can be selected according to the size of the closed space 21, the required resolution, or the like, and is not particularly limited.

(Radio Wave Absorber)

The device for measuring the state of the substance according to the present embodiment irradiates the electromagnetic wave from the transmitting section 22 with respect to the substance 211 inside the closed space 21. For this reason, the electromagnetic wave is repeatedly reflected by the surface of the partition wall of the closed space 21, thereby requiring a long time until the electromagnetic wave is attenuated, and the S/N ratio may deteriorate.

Hence, the device for measuring the state of the substance according to the present embodiment may further include a radio wave absorber 212 inside the closed space 21. A material, shape, and size of the radio wave absorber 212 can be selected according to conditions, such as a wavelength of the electromagnetic wave transmitted from the transmitting section 22, the size of the closed space 21, a temperature inside the closed space 21, or the like, and are not particularly limited.

The radio wave absorber 212 is not limited to an arrangement disposed inside the closed space 21. For example, at least a portion of the wall forming the closed space 21 may be configured to transmit the radio wave (electromagnetic wave), and the radio wave absorber may be disposed outside the closed space 21.

According to the device for measuring the state of the substance according to the present embodiment described above, it is possible to accurately measure the state of the substance inside the closed space, although it was conventionally difficult to perform such a measurement.

(2) Method for Measuring State of Substance

Next, each process of the method for measuring the state of the substance according to the present embodiment will be described. A description of some of the matters already described with respect to the device for measuring the state of the substance will be omitted.

(Irradiating Process)

In the irradiating process (step S11), the electromagnetic wave can be irradiated with respect to the substance 211 inside the closed space 21.

As described above, the closed space 21 is preferably the inside of the heating furnace or the internal combustion engine.

The closed space may further include a radio wave absorber depending on the size of the closed space. The radio wave absorber is not limited to the arrangement disposed inside the closed space. For example, at least a portion of the wall forming the closed space may be configured to transmit the radio wave (electromagnetic wave), and the radio wave absorber may be disposed outside the closed space.

A chirped pulse wave can be used as the electromagnetic wave as described above, and a chirped pulse wave generated by multi-level chirp modulation is preferably used, as required.

In addition, a circularly polarized wave is preferably used as the electromagnetic wave. Because the substance 211 to be measured usually has a three-dimensional shape, the use of the circularly polarized wave makes it possible to also measure the particle size distribution or the like of the substance with a particularly high accuracy, for example.

The circularly polarized wave may be a combination of a plurality of polarized waves, such as a right-handed polarized wave, a left-handed polarized wave, or the like, and the data processing process may analyze the transmitting and received data by the plurality of polarized waves.

(Receiving Process)

In the receiving process (step S12), the electromagnetic wave can be received. Specifically, the electromagnetic wave can be irradiated with respect to the substance in the irradiating process (step S11), and the electromagnetic wave reflected from the substance can be received.

(Data Processing Process)

In the data processing process (step S13), the data processing can be performed on the data of the electromagnetic wave received in the receiving process (step S12).

The content of the data processing performed in the data processing process is not particularly limited, and a data processing corresponding to the data on the state of the substance to be acquired or the like can be performed. For example, the data of the electromagnetic wave received in the receiving process can be separated into a real part and an imaginary part, and the data processing can be performed according to the data on the state of the substance to be acquired.

The data processing process preferably includes a chirp compression. In a case where a chirped pulse wave generated by the multi-level chirp modulation is used as the electromagnetic wave in the irradiating process, the multi-level chirp compression can be performed in the data processing process according to the number of times of the chirp modulation is performed, the chirp rate, or the like. By performing the multi-level chirp compression, it is possible to increase the resolution.

In the data processing process, noise can be removed, and a further data processing can be performed according to the state of the substance to be measured from the data after the chirp compression, as required.

(Delay Processing Process)

The method for measuring the state of the substance according to the present embodiment may include a delay processing process with respect to the received electromagnetic wave, between the receiving process and the data processing process, as required.

The method for measuring the state of the substance according to the present embodiment is a method for measuring the state of the substance inside the closed space. For this reason, there are cases where it is difficult to separate the scattered wave and the direct wave, as described above.

Accordingly, by performing the delay processing process, it is possible to secure a sufficient time after the electromagnetic wave is transmitted from the transmitting section until the electromagnetic wave is converted into the electric signal by the receiver. For this reason, it is possible to duplicate a situation similar to a case where the measurement is performed in a space larger than the closed space in which the measurement is being performed, so that the scattered wave and the direct wave described above can easily be separated.

The delay processing process can be performed using the delay line described above, for example.

According to the method for measuring the state of the substance according to the present embodiment described above, it is possible to accurately measure the state of the substance inside the closed space, although it was conventionally difficult to perform such a measurement.

(3) Others

In the device and method for measuring the state of the substance according to the present embodiment described above, the closed space can be the inside of the heating furnace or the internal combustion engine, as described above. According to the device and method for measuring the state of the substance according to the embodiment, it is possible to measure the state of the substance, which is a material or fuel supplied to the inside of the heating furnace or the internal combustion engine, which is a closed space. The state of the substance refers to a distribution state, a mixed state, a composition, a moisture percentage, a molten state, a viscosity, an adhesion, a repose angle, or the like of the substance inside the closed space.

The device and method for measuring the state of the substance according to the present embodiment may further include, a supply device configured to supply the substance such as a raw material or the like into the closed space, a discharge device configured to discharge the substance processed inside the closed space, a controller, or the like, for example.

The supply device may be a device capable of storing and supplying the raw material or fuel as the substance, such as a hopper, a tank, or the like, and can be connected to the closed space via a pipe or the like. A valve, a pump, a flowmeter, or the like may be provided on the pipe, as required.

The discharge device may be a device capable of discharging the substance processed inside the closed space, and may be a discharge pipe, an exhaust pipe, a tank for storing a product which is the substance processed inside the closed space, or the like, and may be connected to the closed space via a pipe or the like. A valve or the like may be provided on the pipe, as required.

The controller can control the supply device to supply a predetermined amount of the substance or the substance having predetermined characteristics to the closed space, so that an operation state of the heating furnace, the internal combustion engine, or the like including the closed space can be optimized, based on the analysis result of the state of the substance inside the closed space obtained by the analyzer described above.

The controller can also control the operation state of the heating furnace, the internal combustion engine, or the like including the closed space, based on the analysis result of the state of the substance inside the closed space obtained by the analyzer. Specifically, in the case where the closed space is the heating furnace, the controller can also control a heating temperature or the like of the heating furnace.

Moreover, the controller can control a discharge amount and discharge speed of the substance from the closed space with respect to the discharge device, based on the analysis result of the state of the substance inside the closed space obtained by the analyzer described above, and control a residence time of the substance or the like inside the closed space.

The controller may include a CPU, a main storage device, an auxiliary storage device, an input-output interface, or the like so as to perform a data processing on control conditions or the like and communicate with the supply device, the heating furnace or the like including the closed space, the discharge device, or the like. The main storage device may be a RAM or a ROM, and the auxiliary storage device may be an SSD, an HDD, or the like. The input-output interface may be a communication interface for exchanging control signals and data with the supply device, the heating furnace or the like including the closed space, and the discharge device. A type of the communication interface is not particularly limited. Any wired or wireless communication method can be used, and examples thereof include a wired local area network (LAN), a wireless LAN, or the like.

As described above, the amount of substance supplied from the supply device and the operating conditions of the heating furnace or the like including the closed space, the amount of substance discharged by the discharge device, or the like can be controlled, based on the measurement result of the state of the substance inside the closed space obtained by the device and method for measuring the state of the substance according to the present embodiment. For this reason, the operating conditions of the heating furnace or the like including the closed space can be optimized. As a result, in the case where the closed space is the heating furnace, it is possible to improve a heating efficiency, and the characteristics and productivity of the substance obtained by the heating furnace. Further, in the case where the closed space is the internal combustion engine, it is possible to reduce incomplete combustion or the like and optimize the combustion state, thereby improving a fuel efficiency and reducing the amount of exhaust gas.

[Exemplary Implementations]

Next, the present invention will be described in more detail with reference to the following exemplary implementations. However, the present invention is not limited to the following exemplary implementations.

[Exemplary Implementation 1]

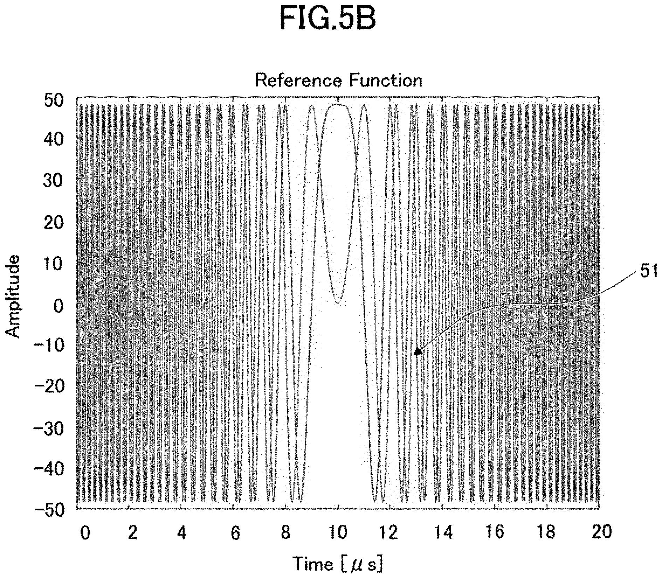

FIG. 5A illustrates an example of a calculation result using a three-dimensional finite difference time domain (FDTD) method related to a scattering state when the electromagnetic wave is irradiated into a closed space surrounded by a perfect conductor having a cylindrical shape with an inner diameter of 1 m and a length of 10 m. The irradiated electromagnetic wave has a center frequency of 5.3 GHz, includes a reference signal 51 illustrated in FIG. 5B, and is a chirped pulse wave which varies from 5.2 GHz to 5.4 GHz, and a substance (object) was placed at a position A having a distance of 2.5 m from a transmitting and receiving antenna 50 inside the closed space. The shape or the like of the substance will be described later. A unit of the numerical values on the coordinate axes in FIG. 5B is a pixel, and in this example, one pixel is a cube having one side of 28.3 mm (0.5 wavelength).

Specifically, the substance inside the closed space was irradiated with the electromagnetic wave from the transmitting section (irradiating process), and the electromagnetic wave was received by the receiving section (receiving process). In the irradiating process, a pulse wave including the reference signal 51 having the waveform illustrated in FIG. 5B as a pulse signal, that is, chirped pulse wave, was used as the electromagnetic wave. As illustrated in FIG. 5B, a pulse width of the chirped pulse wave was 20 us, and a pulse repetition interval 32 (refer to FIG. 3) was 200 $\mu$s. A signal having an output waveform thereof expressed by the Formula (2) described above was used as the pulse signal of the chirped pulse wave. That is, a signal calculated by a formula of the output waveform not including the weight function W(t) was used as the pulse signal. In addition, a constant was used for $\alpha$ in the Formula (2).

A right-handed circularly polarized wave type was used as the transmitting antenna which is a terminating end of the transmitting section, and a left-handed circularly polarized wave type was used as the receiving antenna which is an input end of the receiving section. That is, a circularly polarized wave was used as the electromagnetic wave in the irradiating process.

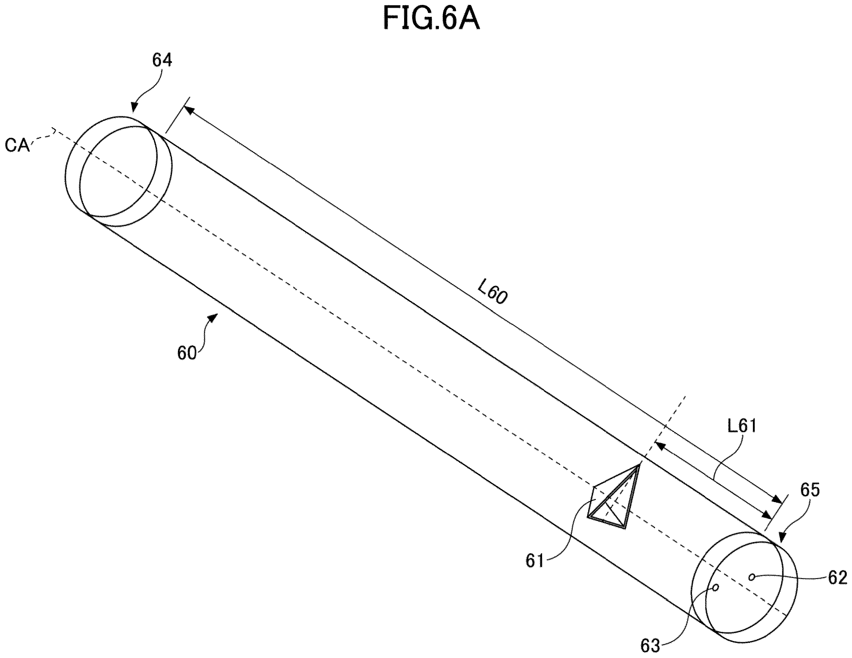

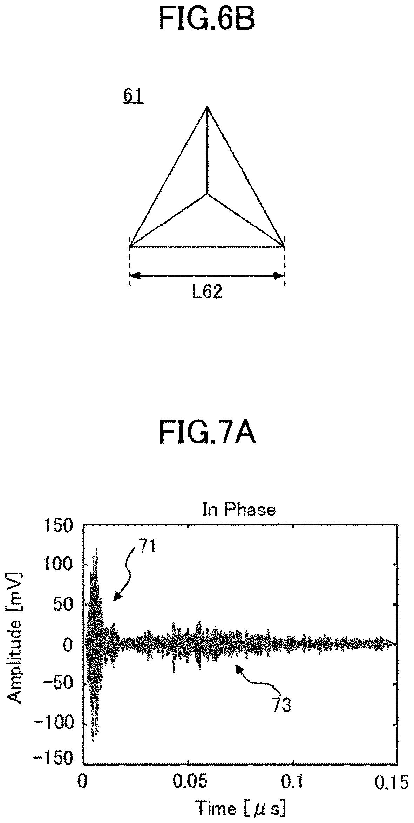

FIG. 6A and FIG. 6B illustrate details of the closed space and the shape and arrangement of the substance which is a target of the electromagnetic scattering state calculation of FIG. 5A and FIG. 5B. As described above and as illustrated in FIG. 6A, a closed space 60 has a cylindrical shape with an inner diameter of 1 m and a length L60 of 10 m, and a substance 61 is disposed inside the closed space 60. FIG. 6B is an enlarged view of the substance 61 and corresponds to a front view of the substance 61. As illustrated in FIG. 6B, the substance 61 was formed into a shape simulating a conical corner reflector formed by joining three triangular plates having a base side with a length L62 of 0.5 m and a vertex angle of 90°. A bottom surface of the substance 61 was arranged to oppose a transmitting antenna 62 and a receiving antenna 63.

The transmitting antenna 62, which is a transmitting section, and the receiving antenna 63, which is a receiving section, are disposed at positions that are approximately 3 cm from a surface of a radio wave absorber 65, and are separated by approximately 20 cm in a horizontal direction from a center axis CA of the cylinder forming the closed space 60. The transmitting antenna 62 and the receiving antenna 63 are connected to the analyzer via the transmitter, the receiver, or the like that are not illustrated. Radio wave absorbers 64 and 65 having a thickness of approximately 30 cm were provided at both ends of a closed space simulating cylinder, and a time progress of the scattering state of the electromagnetic wave irradiated from the transmitting antenna 62 was calculated. An example of the result is illustrated in FIG. 5A.

Further, the analyzer performed a data processing (data processing process) on the electromagnetic wave received by the receiving section in the receiving process. In the analysis of the received signal in the data processing process, a chirp compression was performed.

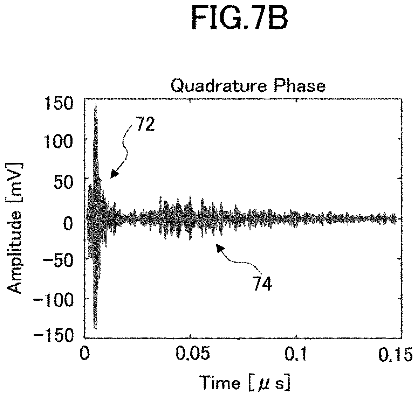

The calculation results of a real part (In-Phase: I) and an imaginary part (Quadrature: Q) of the signal received by the receiving section and the receiver are illustrated in FIG. 7A and FIG. 7B, respectively. If the received signal $g_R(t)$ is expressed as $g_R(t) = \exp(it)$, where i denotes the imaginary unit, a relationship between the real part and the imaginary part of the signal can be expressed by Euler's formula $\exp(it) = \sin(t) + i \cdot \cos(t)$.

In FIG. 7A and FIG. 7B, a signal 71 and a signal 72 indicate directly received waves (direct waves) received when the electromagnetic wave irradiated from the transmitting antenna is scattered by an object other than the nearby object to be measured or directly reaches the receiving antenna without being scattered.

In FIG. 7A and FIG. 7B, a signal 73 and a signal 74 indicate scattered signals from the substance 61.

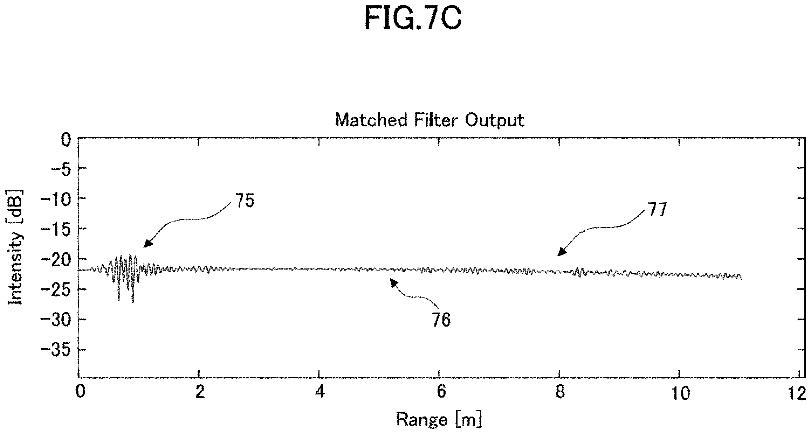

Specifically, as the chirp compression, a correlation processing between the received signal $g_R(t)$ and the chirped signal $g_s$ at the time of transmission is performed according to the Formulas (7) and (8) described above. A signal waveform after the processing is illustrated in FIG. 7C. As may be seen by comparing the signal waveforms after the analysis illustrated in FIG. 7A and FIG. 7B with FIG. 7C, the signal generated by multiple scattering can be separated from the signal from the desired substance by the correlation processing using the chirp compression or the like.

Specifically, as illustrated in FIG. 7C, a direct wave 75, and a scattered wave 76 which is the signal from the desired substance, that is, the object to be measured, can be separated more clearly and received compared to the received signal before the processing, and a signal of multiple scattering 77 can also be separated and received.

As described above, even under an environment of "strong multiple scattering" causing a big problem in the electromagnetic wave scattering analysis inside the closed space, it was possible to separate and receive the desired scattered wave including various information of the object to be measured. Accordingly, it was illustrated that a measurement of the variation in the state of the substance and a continuous observation of the substance position information can be performed, by observing the electromagnetic wave scattering of the various states of a known substance inside the closed space in advance or by using the data obtained immediately before by the continuous measurement as a reference.

[Exemplary Implementation 2]

A conical corner reflector was disposed inside a cylindrical closed space, and the state of the substance inside the closed space was measured by irradiating the electromagnetic wave on the substance.

The closed space of the device for measuring the state of the substance used has a cylindrical shape, similar to the case of the exemplary implementation 1, that is, similar to the closed space 60 illustrated in FIG. 6A. In this exemplary implementation, the inner diameter of the closed space 60 is 35 cm, and the length L60 is 2 m.

Further, the substance 61 was placed inside the closed space 60. The substance 61 was formed in a shape simulating a conical corner reflector formed by joining three triangular plates having a base side with a length L62 of 0.1 m and a vertex angle of 90° (refer to FIG. 6B). The bottom surface of the substance 61 was arranged to oppose the transmitting antenna 62 and the receiving antenna 63.

The cylindrical closed space 60 was formed by wrapping an aluminum foil on a surface of a paper cylinder. However, the aluminum foil was removed from the end of the closed space 60 where the transmitting antenna 62 and the receiving antenna 63 are provided in a range of 150 mm along a longitudinal direction of the closed space 60. Because the closed space 60 is provided in an anechoic chamber, the electromagnetic wave is transmitted through the portion where the aluminum foil is removed, and a wall surface of the anechoic chamber absorbs the electromagnetic wave. That is, the wall surface of the anechoic chamber corresponds to the radio wave absorber. For this reason, the radio wave absorbers 64 and 65 illustrated in the FIG. 6A were not provided.

The transmitting antenna 62, which is the transmitting section, and the receiving antenna 63, which is the receiving section, are provided at the end of the closed space 60, at positions separated by approximately 10 cm in the horizontal direction from the center axis CA of the cylinder forming the closed space 60. The transmitting antenna 62 and the receiving antenna 63 are connected to the analyzer via the transmitter, the receiver, or the like that are not illustrated.

In addition, the substance 61 inside the closed space 60 was irradiated with the electromagnetic wave from the transmitting section (irradiating process), and the electromagnetic wave was received by the receiving section (receiving process). In the irradiating process, a chirped pulse wave having center frequency of 5.4 GHz and a band of 5.0 GHz to 5.8 GHz was used as the electromagnetic wave. The pulse width of the chirped pulse wave was 500 μs, and the pulse repetition interval 32 (refer to FIG. 3) was 1000 μs. A signal having an output waveform thereof expressed by the Formula (2) described above was used as the pulse signal of the chirped pulse wave. That is, a signal calculated by a formula of the output waveform not including the weight function W(t) was used as the pulse signal. Moreover, a constant was used for $\alpha$ in the Formula (2).

A right-handed circularly polarized wave type was used as the transmitting antenna which is the terminating end of the transmitting section, and a right-handed circularly polarized wave type was used as the receiving antenna which is the input end of the receiving section. That is, a circularly polarized wave was used as the electromagnetic wave in the irradiating process.

The position of the substance 61 placed inside the closed space 60 was changed, and the irradiating process and the receiving process were performed for cases where a distance L61 of the substance 61 from the end of the closed space 60 where the receiving antenna 63 or the like are provided is 500 mm and 800 mm, respectively.

Next, the analyzer performed the data processing (data processing process) on the electromagnetic wave received by the receiving section in the receiving process.

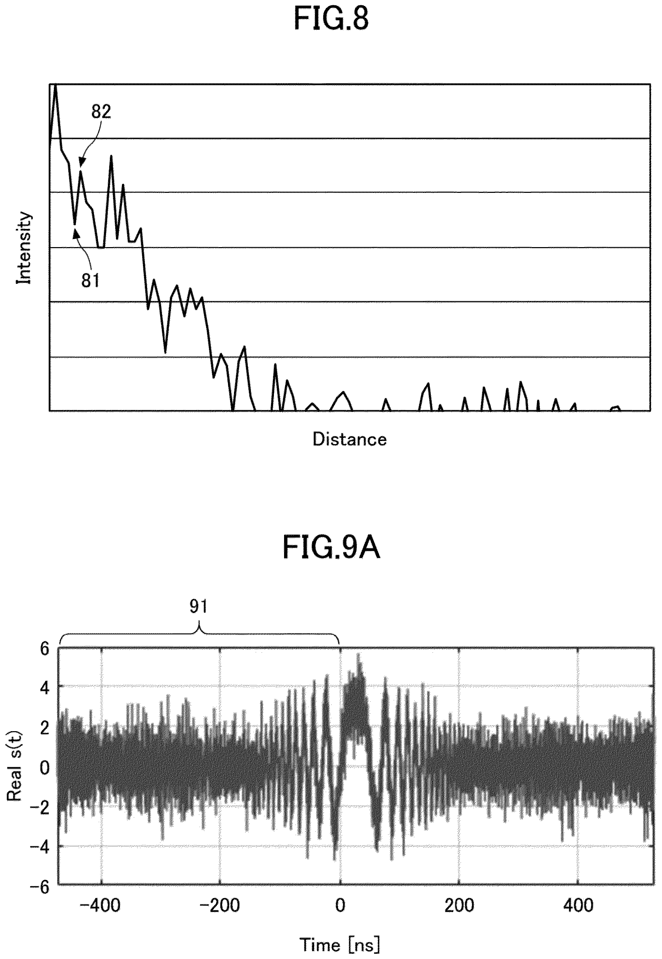

In the data processing process, the data of the substance 61 placed inside the closed space at different positions were subtracted from the signal obtained as an imaginary number in the analyzer, using the Euler's formula described above on the calculation results of the real part (In-Phase: I) and the imaginary part (Quadrature: Q) of the signal received by the receiving section and the receiver. Specifically, differences of real parts and imaginary parts, between the real part and the imaginary part of the signal received for the case where the distance L61 described above is 500 mm and the real part and the imaginary part of the signal received for the case where the distance L61 described above is 800 mm, were obtained, and an absolute value of an imaginary number having a real part and an imaginary part corresponding to the difference between the real parts and the difference between the imaginary parts, respectively, that is, "a square root of a sum of squares of the real part and the imaginary part", was calculated as an intensity. The calculation result is illustrated in FIG. 8.

In FIG. 8, the abscissa corresponds to the distance from the end of the closed space 60 where the transmitting antenna 62 and the receiving antenna 63 are provided. In addition, the ordinate indicates the intensity of the signal described above.

As illustrated in FIG. 8, peaks were confirmed at a point 81 corresponding to the position where the distance L61 is 500 mm, and a point 82 corresponding to the position where the distance L61 is 800 mm. That is, it was confirmed that the state where the substance 61 is disposed at a predetermined position inside the closed space 60 can be measured by the method for measuring the state of the substance and the device for measuring the state of the substance used in this exemplary implementation.

[Exemplary Implementation 3-1 and Exemplary Implementation 3-2]

An exemplary implementation 3-1 and an exemplary implementation 3-2 illustrate examples of a calculation result using a two-dimensional finite difference time domain (FDTD) method related to a scattering state when the electromagnetic wave is irradiated into a closed space surrounded by a perfect conductor having a cylindrical shape.

Because the exemplary implementation 3-1 and the exemplary implementation 3-2 differ only in the chirped pulse wave which is the electromagnetic wave irradiated in the irradiating process and the processing content in the data processing process, a description will first be given of a common configuration.

The closed space of the device for measuring the state of the substance used is assumed to be similar to that of the exemplary implementation 1, that is, assumed to have a cylindrical shape similar to that of the closed space 60 illustrated in FIG. 6A. However, in this exemplary implementation, because the calculation is performed in two dimensions, the closed space becomes a rectangular shape (two-dimensional shape) having a cross section including the center line CA in FIG. 6A. A width of the rectangular shape corresponding to the inner diameter of the closed space 60 is 35 cm, and a length L60 of the rectangular shape is 10 m.

Inside the closed space, the substances 61 (objects) were placed at positions having distances of 3.7 m, 4.0 m, and 4.3 m from the transmitting and receiving antenna 50.

Each of the substances 61 had a shape obtained by removing a base from a triangle having a base with a length L62 of 0.1 m and a vertex angle of 90°, and had a V-shape simulating a corner reflector (refer to FIG. 6B), similar to the exemplary implementation 2. The bottom surface of the substance 61 was arranged to oppose the transmitting antenna 62 and the receiving antenna 63.

The transmitting antenna 62, which is a transmitting section, and the receiving antenna 63, which is a receiving section, are disposed at positions that are approximately 3 cm from the surface of the radio wave absorber 65, and are separated by approximately 10 cm in the horizontal direction from the center axis CA of the closed space 60. The transmitting antenna 62 and the receiving antenna 63 are connected to the analyzer via the transmitter, the receiver, or the like that are not illustrated. Radio wave absorbers 64 and 65 having a thickness of approximately 30 cm were provided at both ends of a rectangle representing a closed space simulating cylinder in two dimensions, and a time progress of the scattering state of the electromagnetic wave irradiated from the transmitting antenna 62 was calculated.

Specifically, the electromagnetic wave from the transmitting section was irradiated with respect to the substance inside the closed space (irradiating process), and the electromagnetic wave was received by the receiving section (receiving process). In the irradiating process, a chirped pulse wave was used as the electromagnetic wave. The pulse width of the chirped pulse wave was 1 us, and the pulse repetition interval 32 (refer to FIG. 3) was 2 us. A right-handed circularly polarized wave type was used as the transmitting antenna which is the terminating end of the transmitting section, and a right-handed circularly polarized wave type was used as the receiving antenna which is the input end of the receiving section. That is, a circularly polarized wave was used as the electromagnetic wave in the irradiating process.

Next, the analyzer performed a data processing (data processing process) on the electromagnetic wave received by the receiving section in the receiving process. In the analysis of the received signal in the data processing process, a chirp compression was performed.

Specifically, as the chirp compression, a correlation processing between the received signal $g_R(t)$ and the chirped signal $g_s$ at the time of transmission is performed according to the Formulas (7) and (8) described above.

In the exemplary implementation 3-1, a signal having an output waveform thereof expressed by the Formula (2) described above was used as the pulse signal of the chirped pulse wave. That is, a signal calculated by a formula of the output waveform not including the weight function $W(t)$ was used as the pulse signal. In addition, a constant was used for $\alpha$ in the Formula (2), not a function of the time t, and $\alpha = 2 \times Bw/Tp$ is set (refer to FIG. 4B), where $Bw = 800$ MHz and $Tp = 1$ $\mu$s.

In the exemplary implementation 3-2, a signal having an output waveform thereof expressed by the Formula (3) described above was used as the pulse signal of the chirped pulse wave. That is, a signal calculated by a formula of the output waveform including the weight function $W(t)$ was used as the pulse signal, and the Kaiser window expressed by the Formula (4) was used as the weight function $W(t)$. In the exemplary implementation 3-2, $\beta$ in the Formula (4) was set to 1.0. In addition, in the exemplary implementation 3-2, $\alpha$ in the Formula (3) was set to a function of the time t represented by the Formula (5) and the Formula (6), and m in the Formula (6) was set to 0.6. Moreover, Bw and Tp were the same as those in the exemplary implementation 3-1.

For this reason, in the exemplary implementation 3-2, a chirped pulse wave generated by multi-level chirp modulation was used as the electromagnetic wave in the irradiating process. In the data processing process, a multi-level chirp compression was performed in accordance with the above.

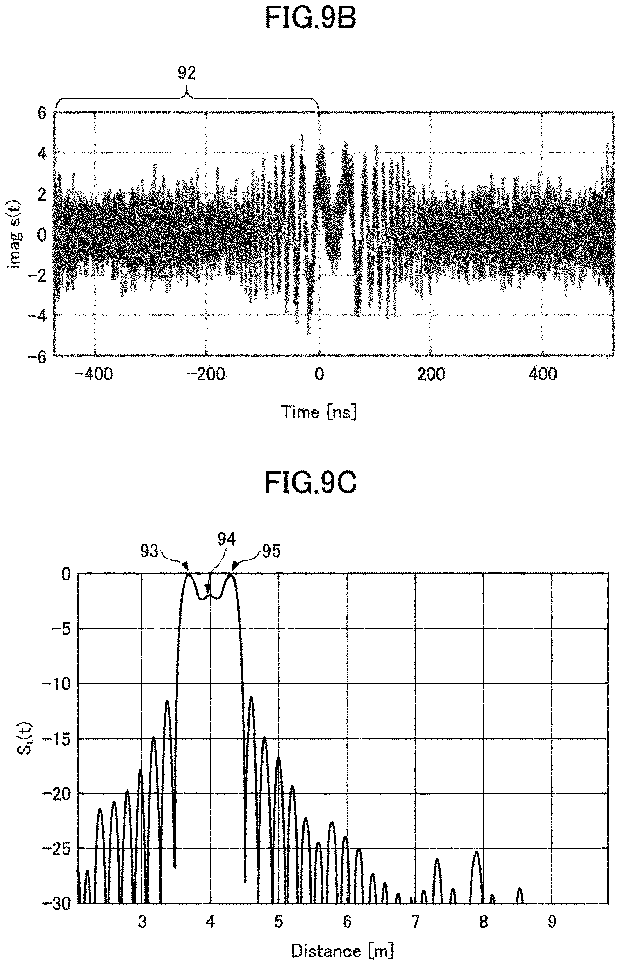

The calculation result of the real part of the signal obtained in exemplary implementation 3-1 is illustrated in FIG. 9A, and the calculation result of the imaginary part of the signal obtained in exemplary implementation 3-1 is illustrated in FIG. 9B. The reason why the time t in regions 91 and 92 has negative values in the FIG. 9A and FIG. 9B is because the delay line is used.

The results of analyzing the signal obtained in the exemplary implementation 3-1 are illustrated in FIG. 9C. As illustrated in FIG. 9C, peaks 93, 94, and 95 were observed at positions having distances of 3.7 m, 4.0 m, and 4.3 m from the transmitting antenna. That is, it was confirmed that three corner reflectors provided inside the closed space 60 can be detected.

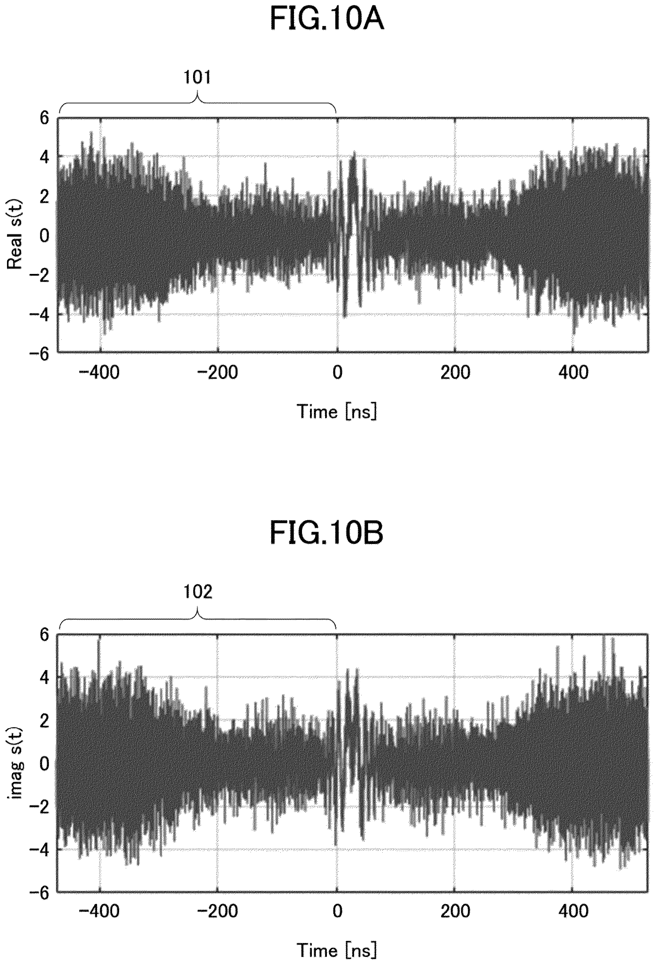

The calculation result of the real part of the signal obtained in the exemplary implementation 3-2 is illustrated in FIG. 10A, and the calculation result of the imaginary part of the signal obtained in the exemplary implementation 3-2 is illustrated in FIG. 10B. The reason why the time t in regions 101 and 102 has negative values in the FIG. 10A and FIG. 10B is because the delay line is used.

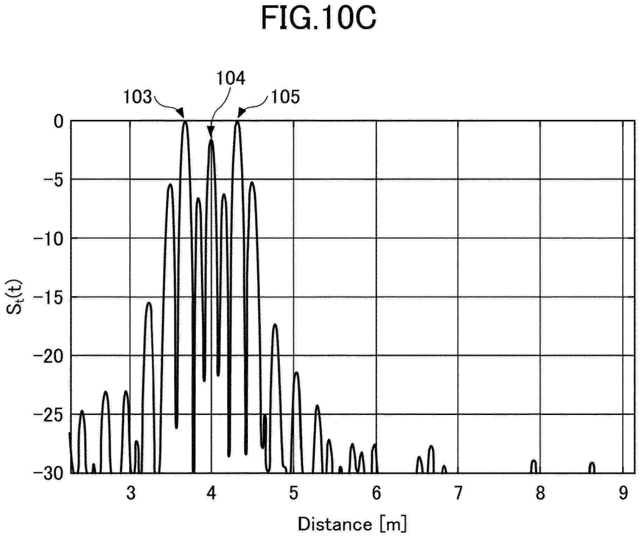

The results of analyzing the signal obtained in the exemplary implementation 3-2 are illustrated in FIG. 10C. As illustrated in FIG. 10C, peaks 103, 104, and 105 were observed at positions having distances of 3.7 m, 4.0 m, and 4.3 m from the transmitting antenna. That is, it was confirmed that three corner reflectors provided inside the closed space 60 can be detected.

From a comparison between the analysis results of the exemplary implementation 3-1 illustrated in FIG. 9C and the analysis results of the exemplary implementation 3-2 illustrated in FIG. 10C, it was confirmed that the analysis results of the exemplary implementation 3-2 can more clearly identify the three corner reflectors. It may be regarded that the exemplary implementation 3-2 can more clearly identify the three corner reflectors, because the chirped pulse wave generated by the multi-level chirp modulation was used as the electromagnetic wave in the irradiating process, and the multi-level chirp compression was performed in the data processing process in correspondence with the use of the chirped pulse wave.

The method for measuring the state of the substance and the device for measuring the state of the substance are described above in the embodiments or the like, but the present invention is not limited to the embodiments or the like. Various variations and modifications can be made without departing from the scope of the subject matter of the present invention described in the claims.

This application is based upon and claims priority to Japanese Patent Application No. 2021-196068 filed with the Japan Patent Office on December 2, 2021, the entire contents of which are incorporated herein by reference.

DESCRIPTION OF REFERENCE NUMERALS

S11: Irradiating process
S12: Receiving process

S13: Data processing process
20: Device for measuring state of substance
21, 60: Closed space
211, 61: Substance
212, 64, 65: Radio wave absorber
22: Transmitting section
23: Receiving section
29: Analyzer

## Claims

1. A method for measuring a state of a substance, the method comprising:

   an irradiating process that irradiates an electromagnetic wave with respect to the substance inside a closed space;
   a receiving process that receives the electromagnetic wave; and
   a data processing process that performs a data processing on the electromagnetic wave received by the receiving process,
   wherein the irradiating process uses a chirped pulse wave as the electromagnetic wave.

2. The method for measuring the state of the substance as claimed in claim 1, wherein the irradiating process uses a circularly polarized wave as the electromagnetic wave.

3. The method for measuring the state of the substance as claimed in claim 1 or 2, wherein the data processing process performs a chirp compression.

4. The method for measuring the state of the substance as claimed in claim 3, wherein:

   the irradiating process uses a chirped pulse wave generated by a multi-level chirp modulation as the electromagnetic wave, and
   the data processing process performs a multi-level chirp compression.

5. The method for measuring the state of the substance as claimed in any one of claims 1 to 4, wherein a radio wave absorber is provided inside the closed space.

6. The method for measuring the state of the substance as claimed in any one of claims 1 to 5, further comprising:
   a delay processing process that performs a delay process on the received electromagnetic wave between the receiving process and the data processing process.

7. The method for measuring the state of the substance as claimed in any one of claims 1 to 6, wherein the closed space is an inside of a heating furnace or an internal combustion engine.

8. A device for measuring a state of a substance, the device comprising:

   a closed space;
   a transmitting section configured to irradiate an electromagnetic wave with respect to the substance inside the closed space;
   a receiving section configured to receive the electromagnetic wave; and
   a data analyzer configured to perform a data processing on the electromagnetic wave received by the receiving section,
   wherein the transmitting section uses a chirped pulse wave as the electromagnetic wave.

9. The device for measuring the state of the substance as claimed in claim 8, wherein the transmitting section uses a circularly polarized wave as the electromagnetic wave.

10. The device for measuring the state of the substance as claimed in claim 8 or 9, wherein the analyzer performs a chirp compression.

**11.** The device for measuring the state of the substance as claimed in claim 10, wherein:

the transmitting section uses a chirped pulse wave generated by a multi-level chirp modulation as the electromagnetic wave, and
the analyzer performs a multi-level the chirp compression.

**12.** The device for measuring the state of the substance as claimed in any one of claims 8 to 11, further comprising:
a radio wave absorber provided inside the closed space.

**13.** The device for measuring the state of the substance as claimed in any one of claims 8 to 12, further comprising:
a delay line provided between the receiving section and a receiver.

**14.** The device for measuring the state of the substance as claimed in any one of claims 8 to 13, wherein the closed space is an inside of a heating furnace or an internal combustion engine.

# FIG.1

10

```
        ┌──────────────┐
        │    START     │
        └──────┬───────┘
               │
               ▼
   ┌───────────────────────┐
   │  IRRADIATING PROCESS  │───S11
   └───────────┬───────────┘
               │
               ▼
   ┌───────────────────────┐
   │   RECEIVING PROCESS   │───S12
   └───────────┬───────────┘
               │
               ▼
   ┌───────────────────────┐
   │ DATA PROCESSING PROCESS │───S13
   └───────────┬───────────┘
               │
               ▼
        ┌──────────────┐
        │     END      │
        └──────────────┘
```

# FIG.2

EP 4 443 145 A1

# FIG.3

EP 4 443 145 A1

# FIG.4A

# FIG.4B

# FIG.4C

# FIG.4D

# FIG.4E

# FIG.5A

# FIG.5B

Reference Function

FIG.6A

# FIG.6B

61

L62

# FIG.7A

In Phase

Amplitude [mV] vs Time [$\mu$s]

71

73

# FIG.7B

Quadrature Phase

# FIG.7C

Matched Filter Output

# FIG.8

# FIG.9A

# FIG.9B

# FIG.9C

# FIG.10A

# FIG.10B

# FIG.10C

## INTERNATIONAL SEARCH REPORT

| International application No. |
|---|
| **PCT/JP2022/044265** |

### A. CLASSIFICATION OF SUBJECT MATTER

***G01N 22/00***(2006.01)i; ***G01N 22/04***(2006.01)i
FI: G01N22/00 N; G01N22/00 S; G01N22/00 R; G01N22/04 Z

According to International Patent Classification (IPC) or to both national classification and IPC

### B. FIELDS SEARCHED

Minimum documentation searched (classification system followed by classification symbols)

G01N22/00-22/04; G01V3/12; G01S13/00-13/95

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2020-3506 A (KAWASAKI GEOLOGICAL ENG CO LTD) 09 January 2020 (2020-01-09) claims 1-2, paragraphs [0020]-[0033], fig. 2, 6, 12 | 1, 3, 6, 8, 10, 13 |
| Y | | 2, 4-5, 7, 9, 11-12, 14 |
| X | CN 112557373 A (NANJING UNIVERSITY OF SCIENCE AND TECHNOLOGY) 26 March 2021 (2021-03-26) paragraphs [0041], [0043], fig. 2 | 1, 8 |
| Y | | 2-7, 9-14 |
| Y | JP 4-27511 B2 (NIPPON TELEGRAPH & TELEPHONE) 12 May 1992 (1992-05-12) claim 1 | 2-7, 9-14 |
| Y | JP 2002-350364 A (TOSHIBA IT & CONTROL SYSTEMS CORP) 04 December 2002 (2002-12-04) caim 1 | 2-7, 9-14 |
| Y | JP 2002-82162 A (FUJITSU LTD) 22 March 2002 (2002-03-22) caim 1 | 3-7, 10-14 |

☑ Further documents are listed in the continuation of Box C.　　☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **03 February 2023** | **21 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| --- |
| **PCT/JP2022/044265** |

| C. | DOCUMENTS CONSIDERED TO BE RELEVANT | |
| --- | --- | --- |
| Category* | Citation of document, with indication, where appropriate, of the relevant passages | Relevant to claim No. |
| Y | JP 2-27286 A (HITACHI SHONAN DENSHI CO LTD) 30 January 1990 (1990-01-30)<br>p. 2, upper right column, lines 5-8, fig. 5 | 4-7, 11-14 |
| Y | JP 2018-179914 A (DENSO TEN LTD) 15 November 2018 (2018-11-15)<br>claim 1, fig. 4, 5A | 4-7, 11-14 |
| Y | JP 2004-212367 A (NTT DOCOMO INC) 29 July 2004 (2004-07-29)<br>claim 2, fig. 3 | 5-7, 12-14 |
| Y | JP 2013-250263 A (KOREA INSTITUTE OF SCIENCE AND TECHNOLOGY) 12 December 2013 (2013-12-12)<br>claims 13-14 | 6-7, 13-14 |
| Y | JP 8-292163 A (MITSUBISHI HEAVY IND LTD) 05 November 1996 (1996-11-05)<br>claim 1, fig. 1 | 7, 14 |
| Y | JP 3-205543 A (NIPPON SOKEN INC) 09 September 1991 (1991-09-09)<br>claim 1, fig. 1 | 7, 14 |

Form PCT/ISA/210 (second sheet) (January 2015)

**INTERNATIONAL SEARCH REPORT**
Information on patent family members

International application No.

**PCT/JP2022/044265**

| Patent document cited in search report | | | Publication date (day/month/year) | Patent family member(s) | | | Publication date (day/month/year) |
|---|---|---|---|---|---|---|---|
| JP | 2020-3506 | A | 09 January 2020 | (Family: none) | | | |
| CN | 112557373 | A | 26 March 2021 | (Family: none) | | | |
| JP | 4-27511 | B2 | 12 May 1992 | (Family: none) | | | |
| JP | 2002-350364 | A | 04 December 2002 | (Family: none) | | | |
| JP | 2002-82162 | A | 22 March 2002 | (Family: none) | | | |
| JP | 2-27286 | A | 30 January 1990 | (Family: none) | | | |
| JP | 2018-179914 | A | 15 November 2018 | (Family: none) | | | |
| JP | 2004-212367 | A | 29 July 2004 | (Family: none) | | | |
| JP | 2013-250263 | A | 12 December 2013 | US 2013/0321620 A1 claims 13-14 KR 10-2013-0135016 A CN 103454288 A | | | |
| JP | 8-292163 | A | 05 November 1996 | (Family: none) | | | |
| JP | 3-205543 | A | 09 September 1991 | (Family: none) | | | |

Form PCT/ISA/210 (patent family annex) (January 2015)

**REFERENCES CITED IN THE DESCRIPTION**

**Patent documents cited in the description**

- JP 2019183202 A
- JP 2021196068 A